# NEUE EUROPÄISCHE PATENTSCHRIFT

(11) **EP 2 279 715 B2**
(45) Veröffentlichungstag und Bekanntmachung des Hinweises auf die Entscheidung über den Einspruch: **06.09.2023**
(45) Hinweis auf die Patenterteilung: 13.09.2017
(21) Anmeldenummer: 10006017.7
(22) Anmeldetag: 22.04.2005
(51) Int. Cl.: A61F 13/56, A61F 13/64

(54) **ABSORBIERENDER INKONTINENZARTIKEL**
ABSORBENT INCONTINENCE ARTICLE
ARTICLE ABSORBANT POUR INCONTINENTS

(30) Priorität: 23.04.2004 DE 102004021353
(43) Veröffentlichungstag der Anmeldung: 02.02.2011
(62) Teilanmeldung aus: 05744535.5
(73) Patentinhaber: Paul Hartmann AG, 89522 Heidenheim (DE)
(72) Erfinder: Benning, Markus, 89547 Gerstetten (DE); Kesselmeier, Rüdiger, 89542 Herbrechtingen (DE); Koch, Christian, 89429 Bachhagel (DE); Röhrl, Wolfgang, 89542 Herbrechtingen (DE)
(74) Vertreter: DREISS Patentanwälte PartG mbB

(56) Entgegenhaltungen:
- EP-A- 1 269 949
- EP-A1- 1 110 529
- EP-A2- 1 166 735
- WO-A1-02/22062
- WO-A1-86/04812
- WO-A1-98/35641
- WO-A1-99/22687
- DE-A1- 19 732 499
- GB-A- 1 441 567
- US-A1- 2001 023 341
- US-A1- 2001 034 512
- US-B2- 6 626 881

## Beschreibung

Die vorliegende Erfindung betrifft einen absorbierenden Inkontinenzartikel, insbesondere für inkontinente Erwachsene, mit einem Hauptteil, bestehend aus einem Vorderbereich, einem Rückbereich und einem in Längsrichtung dazwischen liegenden zwischen den Beinen eines Benutzers zu liegen kommenden Schrittbereich, wobei der Hauptteil einen Saugkörper umfasst, und mit an den Rückbereich angefügten Materialabschnitten, welche sich in Querrichtung oder Hüftumfangsrichtung über seitliche Längsränder des Hauptteils hinaus erstrecken und den Vorderbereich und den Rückbereich im angelegten Zustand des Artikels miteinander verbinden, wobei diese Materialabschnitte wenigstens um eine in Längsrichtung verlaufende Falzlinie auf sich selbst gefaltet sind.

Bei derartigen Inkontinenzartikeln können die erwähnten, insbesondere nur an den Rückbereich angefügten Materialabschnitte aus einem anderen Material gebildet sein als der Hauptteil oder eine Komponente des Hauptteils, beispielsweise ein flüssigkeitsundurchlässiges Backsheet oder ein flüssigkeitsdurchlässiges Topsheet. Beispielsweise können die Seitenteile bildenden Materialabschnitte, die häufig auch als "Ohren" des Inkontinenzartikels bezeichnet werden, atmungsaktiv, insbesondere luft- und wasserdampfdurchlässig ausgebildet werden, wohingegen der Hauptteil, der häufig auch als Chassis bezeichnet wird, flüssigkeitsundurchlässig, insbesondere feuchtigkeitsundurchlässig ausgeführt sein kann. Zum Schließen des Inkontinenzartikels werden die vorzugsweise unlösbar am Rückbereich angefügten Seitenteile bildenden Materialabschnitte auf die Bauchseite des Benutzers geschlagen und dort mit dem Hauptteil, vorzugsweise mit der körperabgewandten Außenseite des Vorderbereichs des Hauptteils lösbar verbunden. Häufig kommen hierfür mechanisch wirkende oder klebend wirkende Verschlusselemente an den Seitenteilen des Inkontinenzartikels zum Einsatz, die dann mit entsprechend ausgebildeten Auftreffbereichen im Vorderbereich des Hauptteils zusammenwirken. EP 1 269 949 A2 zeigt keinen Inkontinenzartikel, bei dem die von einem Rückbereich vorstehenden Seitenteile an dem Vorderbereich festgelegt werden, sondern eine sogenannte Gürtelwindel, bei der die vom Rückbereich vorstehenden Seitenteile zur Bildung einer in Umfangsrichtung durchgehend geschlossenen Hüftöffnung miteinander verbunden werden. Die den Hüftgürtel bildenden seitlich vom Rückbereich vorstehenden Seitenteile sind Z-förmig aufeinandergefaltet und in dieser Konfiguration nur temporär aneinander fixiert, also nur während der Herstellung des Artikels innerhalb der Maschine. Ähnliche Inkontinenzartikel werden auch in US2001/034512 A1, EP1110529 A1 und US6626881 B2 offenbart. Der vorliegenden Erfindung liegt die Aufgabe zugrunde, ausgehend von einem absorbierenden Inkontinenzartikel der eingangs beschriebenen Art mit verhältnismäßig breiten an den Hauptteil angefügten Materialabschnitten einerseits die Handhabung dieser Materialabschnitte während der Herstellung in einer schnelllaufenden Maschine zu verbessern und andererseits die Handhabung des Inkontinenzartikels bei der Ingebrauchnahme durch den Benutzer oder durch Pflegepersonen möglichst bedienerfreundlich auszugestalten.

Diese Aufgabe wird erfindungsgemäß gelöst durch einen absorbierenden Inkontinenzartikel mit den Merkmalen des Anspruchs 1.

Während bei Gürtelwindeln der Hüftgürtel verhältnismäßig schmal ausgebildet ist, werden absorbierende Inkontinenzartikel der eingangs genannten Art mit recht breiten seitlichen Materialabschnitten gewünscht. Mit der vorliegenden Erfindung wurde ein solcher Inkontinenzartikel geschaffen, bei dem die aufeinander gefalteten Materialabschnitte so lösbar aneinander fixiert sind, dass alle Fügestellen oder Fügebereiche durch einmaliges Ziehen an einem jeweiligen Anfassbereich aufgetrennt werden. Hierdurch wird der Inkontinenzartikel bedienungsfreundlich und erweist sich insbesondere bei der Anwendung bei stark pflegebedürftigen Personen als besonders vorteilhaft. So wird der Inkontinenzartikel beispielsweise häufig an pflegebedürftige Patienten angelegt, während diese sich in Seitenlage befinden. Hierbei muss einer der über den Hauptteil seitlich überstehenden Materialabschnitte unter dem Patienten hindurch geführt werden. Dieser Vorgang des unter dem Patienten Hindurchführens ist mit erfindungsgemäß lösbar fixierten Materialabschnitten nun wesentlich vereinfacht.

Im einfachsten Fall ist ein jeweiliger Materialabschnitt um eine Falzlinie auf sich selbst gefaltet, so dass zwei Teilabschnitte aufeinander liegen bzw. gegeneinander anliegen. Vorzugsweise ist der Materialabschnitt aber um wenigstens zwei Falzlinien auf sich selbst gefaltet, so dass eine im Schnitt Z-förmige Konfiguration entsteht. Nach einer weiteren bevorzugten Ausführungsform sind die Materialabschnitte um drei Falzlinien auf sich selbst gefaltet. Nach einer weiteren bevorzugten Ausführungsform sind die Materialabschnitte um vier Falzlinien auf sich selbst gefaltet.

Nach einer weiteren bevorzugten Ausführungsform des erfindungsgemäßen Inkontinenzartikels sind die jeweiligen Anfassbereiche vor der Entfaltung der Materialabschnitte in Querrichtung nach außen gewandt, also voneinander und von einer Längsmittelachse des auf einer ebenen Unterlage ausgebreiteten Windelhauptteils voneinander weggewandt, so dass sie auf bequeme Weise mit der linken Hand eines Benutzers von links und mit der rechten Hand von rechts ergreifbar sind.

Die lösbare Fixierung der aufeinander gefalteten Teilabschnitte der Materialabschnitte aneinander und möglicherweise auch mit dem Hauptteil ist durch mehrere im Wesentlichen punktförmige Fügestellen ausgebildet. Eine punktförmige Fügestelle der vorstehend erwähnten Art bedeutet, dass die Fügestelle eine Fläche (in Projektion auf die X-Y-Ebene des Hauptteils) von weniger als 5 mm², insbesondere von weniger als 2 mm² und weiter insbesondere von weniger als 1 mm² aufweist. Die Fügestellen müssen nicht streng punkt- oder kreisförmig sein. Denkbar und vorteilhaft sind auch von der Punkt- oder Kreisform abweichende Formen wie Dreieck-, Viereck-, Vieleck-, oder Ovalformen. Die lösbare Fixierung der aufeinander gefalteten Teilabschnitte der Materialabschnitte aneinander ist durch thermisch oder durch Ultraschall erzeugte, punktförmige Fügestellen ausgebildet.

Es wurde erfindungsgemäß erkannt, dass die Anzahl, die Verteilung oder der Flächenanteil der Fügestellen oder die Haftkraft der lösbar aneinander gefügten Teilabschnitte so gewählt werden kann, dass die lösbare Fixierung an sämtlichen Fügestellen oder Fügebereichen beim Entfalten durch einmaliges Ziehen an dem jeweiligen Anfassbereich der Materialabschnitte auftrennbar ist. Dies kann in vorteilhafter Weise dadurch unterstützt werden, dass die Anzahl oder der Flächenanteil der Fügestellen oder die Haftkraft der lösbar aneinander gefügten Teilabschnitte mit der Entfernung von dem Anfassbereich des Materialabschnitts abnimmt. Es wurde erfindungsgemäß erkannt, dass je weiter ein Bereich der aufeinander gefalteten Teilabschnitte der Materialabschnitte von dem Anfassbereich entfernt ist, desto geringer die Stärke der Fixierung der Teilabschnitte aneinander sein sollte, um eine Lösung sämtlicher Fügestellen oder -bereiche durch einmaliges Ziehen an dem jeweiligen Anfassbereich der Materialabschnitte, also durch eine einmalige Entfaltungsbewegung, zu erreichen. Es wurde demzufolge auch erkannt, dass in der Nähe des Anfassbereichs die lösbare Fixierung der aufeinander gefalteten Teilabschnitte problemlos den Anforderungen entsprechend stark ausgebildet werden kann. So kann ein sicherer Transport der vorzugsweise schon vor oder in der schnelllaufenden Windelherstellungsmaschine gefalteten Flachmaterialbahnen gewährleistet werden, ohne dass vom Hauptteil Inkontinenzartikels seitlich vorstehende Materialabschnitte flattern oder aufeinander gefaltete Teilabschnitte innerhalb der Faltung verschoben werden. Es ergibt sich auch später bei der Faltung des gesamten Produkts ein sauberes Erscheinungsbild.

In weiterer Ausbildung der Erfindung erweist es sich als vorteilhaft, wenn in einem Umkreis von 1,5 cm von einem vom Anfassbereich in Ebenenrichtung der gefalteten Konfiguration am weitesten entfernten Punkt der vorzugsweise am weitesten entfernten Falzlinie die aneinander anliegenden Teilabschnitte nicht aneinander gefügt sind. Bei diesem am weitesten entfernten Punkt wird es sich um einen am freien Rand der Materialabschnitte liegenden Punkt der Falzlinie handeln. Doch auch über die gesamte Erstreckung der betrachteten insbesondere am weitesten vom Anfassbereich entfernten Falzlinie erweist es sich als vorteilhaft, wenn in einem Abstand von 5 mm, insbesondere von 8 mm und weiter insbesondere von 10 mm von dieser Falzlinie entfernt die aneinander liegenden Teilabschnitte nicht aneinander gefügt sind.

Es erweist sich des Weiteren als vorteilhaft, wenn die flächenhafte Erstreckung der aufeinander gefalteten und aneinander anliegenden Teilabschnitte (gedanklich) durch eine in Längsrichtung verlaufende Gerade in zwei annähernd gleiche Hälften teilbar ist und wenn die Anzahl oder der Flächenanteil der Fügestellen oder -bereiche oder die Haftkraft der lösbar aneinander gefügten Teilabschnitte in diesen zwei Hälften unterschiedlich ist. Es erweist sich dabei als besonders vorteilhaft, wenn die Anzahl oder der Flächenanteil der Fügestellen oder die Haftkraft der lösbar aneinander gefügten Teilabschnitte in der dem Anfassbereich in Querrichtung zugewandten Hälfte, also der daran angrenzenden Hälfte, größer ist als in der dem Anfassbereich in Querrichtung abgewandten Hälfte.

Die dem Hauptteil des Inkontinenzartikels angefügten Materialabschnitte sind, wie eingangs bereits erwähnt, breiter (quer zur Hüftumfangsrichtung) ausgebildet als dies bei typischen Gürtelwindeln der Fall ist. Die Breite, also die Erstreckung eines Materialabschnitts in Längsrichtung des Hygieneartikels beträgt im Bereich der Anfügung an den Hauptteil vorzugsweise wenigstens 10 cm, insbesondere wenigstens 14 cm, insbesondere wenigstens 18 cm und weiter insbesondere wenigstens 22 cm.

Die Erstreckung eines an den Hauptteil angefügten Materialabschnitts im entfalteten Zustand in Querrichtung über den Längsrand des Hauptteils hinaus, was der Hüftumfangsrichtung im angelegten Zustand entspricht, beträgt wenigstens 10 cm, insbesondere wenigstens 15 cm, und weiter insbesondere wenigstens 18 cm. Sie beträgt vorzugsweise höchstens 35 cm, insbesondere höchstens 30 cm und weiter insbesondere höchstens 27 cm.

Zum Schließen des Inkontinenzartikels im angelegten Zustand an einen Benutzer weisen die Materialabschnitte Verschlusselemente auf, die mechanisch haftend oder klebend ausgebildet sein können, und die vorzugsweise ihrerseits in einer gefalteten, zum Gebrauch entfaltbaren Konfiguration an den Materialabschnitten angeordnet sind. Es erweist sich als zweckmäßig, wenn die Materialabschnitte im Rückbereich solche Verschlusselemente aufweisen, die mit einem Auftreffbereich am Windelhauptteil lösbar haftend oder klebend zusammenwirken können.

Es erweist sich auch als vorteilhaft, wenn die beidseits vorgesehenen Materialabschnitte im Rückbereich eines vor der Ingebrauchnahme zusammengefalteten Inkontinenzartikels auf die körperzugewandte Seite des Hauptteils eingeschlagen sind. Dabei können die eingeschlagenen Materialabschnitte einander teilweise überlappen, wobei aber dennoch deren Anfassbereiche zugleich ergreifbar sind, also visuell wahrnehmbar und manuell greifbar vorgesehen sind.

Die an den Hauptteil angefügten Materialabschnitte sind vorzugsweise aus einem Vliesstoff gebildet, insbesondere und vorzugsweise können Spunbond-Materialien (S) oder Spunbond-Meltblown-Materialien (SM), oder beidseitig mit Spunbond-Materialien versehene Meltblown-Schichten (SMS) oder auch kardierte Vliesmaterialien Verwendung finden. Auch Vliesstofflaminate, also insbesondere zweilagige, dreilagige oder mehrlagige Kombinationen der vorgenannten Vliesstoffe, können Verwendung finden. Die Verbindung der einzelnen Lagen kann durch an sich übliche und bekannte Verfahren, beispielsweise durch thermische Fügeverfahren (Verschweißung, insbesondere Laserverschweißung, hotmelt, air-through) oder durch Ultraschallschweißverfahren erfolgen; auch die kalte Verpressung, Vernadelung, Vernähung oder Verklebung von Vliesstoffmaterialien ist denkbar. Auch die Verbindung mit textilen Geweben, Gewirken oder Gestricken, also mit eine textile Bindung im weitesten Sinne aufweisenden Materialien ist denkbar. Vorzugsweise werden die seitlich an den Hauptteil angefügten Materialabschnitte zumindest abschnittsweise atmungsaktiv ausgebildet, wobei insbesondere eine Mikroporosität als vorteilhaft angesehen wird, die sowohl einen Luftaustausch als auch eine Durchlässigkeit für Feuchtigkeit in Form von Wasserdampf gestattet. Die Materialabschnitte haben in vorteilhafter Weise ein Flächengewicht von 10 bis 150 g/m², insbesondere 20 - 100 g/m², weiter insbesondere 25 - 50 g/m².

Die an den Hauptteil angefügten Materialabschnitte können nach einer weiteren Ausführungsform der Erfindung auch so ausgebildet werden, dass sie weniger steif sind als der Hauptteil oder chassisbildende Materialien des Hauptteils, wie insbesondere das Backsheet oder ein aus Backsheet und Topsheet bestehendes Laminat des Hauptteils. Auf diese Weise kann ein hautfreundlicher Seitenabschnitt des Hygieneartikels erreicht werden, der vorzugsweise textil bzw. vliesartig anmutet und vom Benutzer als angenehm empfunden wird.

Es erweist sich nach einem an sich selbständigen Erfindungsgedanken als besonders vorteilhaft, dass die Fügestellen oder Fügebereiche beim Entfalten der Materialabschnitte durch Ziehen an dem jeweiligen Anfassbereich eine über den Entfaltungsvorgang gemittelte Spitzenkraft von höchstens 2,5 N, insbesondere von höchstens 2,4 N, insbesondere von höchstens 2,3 N, insbesondere von höchstens 2,2 N, insbesondere von höchstens 2,1 N und weiter insbesondere von höchstens 2,0 N entgegensetzen. Ermittelt man während eines Entfaltungsvorgangs durch Ziehen an dem jeweiligen Anfassbereich die dabei in jedem Zeitpunkt auftretende Zugkraft und berücksichtigt diejenigen Kraftspitzen, die sich von unmittelbar angrenzenden, benachbarten Bereichen um wenigstens 0,5 N unterscheiden, so ist es möglich, sämtliche so ermittelten Kraftspitzen während eines Entfaltungsvorgangs zu mitteln, also eine über die Anzahl dieser Kraftspitzen gemittelte Spitzenkraft zu errechnen.

Berücksichtigt man nicht nur einen Entfaltungsvorgang, sondern sechs Entfaltungsvorgänge von sechs identisch gebildeten und gefalteten Materialabschnitten und mittelt die wie vorausgehend ermittelten gemittelten Spitzenkräfte über die sechs Entfaltungsvorgänge, so betragen diese höchstens 2,0 N, insbesondere von höchstens 1,8 N, insbesondere von höchstens 2,3 N, insbesondere von höchstens 1,7 N, insbesondere von höchstens 1,6 N und weiter insbesondere von höchstens 1,5 N entgegensetzen.

Es erweist sich des Weiteren als besonders vorteilhaft, dass nach einem weiteren an sich unabhängigen Erfindungsgedanken die Materialabschnitte derart gefaltet und vorfixiert sind, dass die beim Entfalten eines Materialabschnitts in einem Zug erforderliche Arbeit gemittelt über sechs Entfaltungsvorgänge höchstens 120 Nmm, insbesondere höchstens 115 Nmm, insbesondere höchstens 110 Nmm, insbesondere höchstens 105 Nmm, insbesondere höchstens 100 Nmm, insbesondere höchstens 95, insbesondere höchstens 90 Nmm beträgt.

Es wird nachfolgend ein Test zur Bestimmung der beim Entfalten der auf sich selbst gefalteten Materialabschnitte zu überwindenden Kräfte und zur Prüfung des Entfaltens der Materialabschnitte in einem Zug beschrieben. Es wird unter Verwendung eines Zugprüfgeräts nach EN ISO 527-1 (April 1996) die in jedem Moment auftretende Kraft über den Öffnungsweg ermittelt und aufgezeichnet.

### Probenvorbereitung:

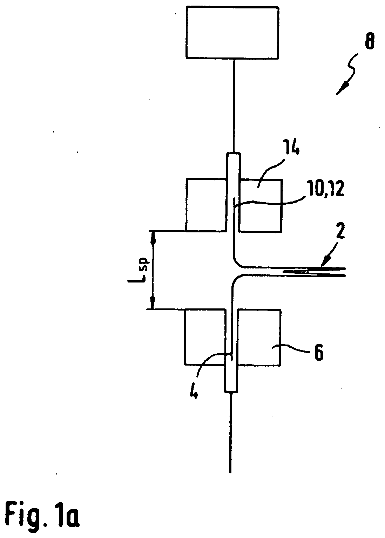

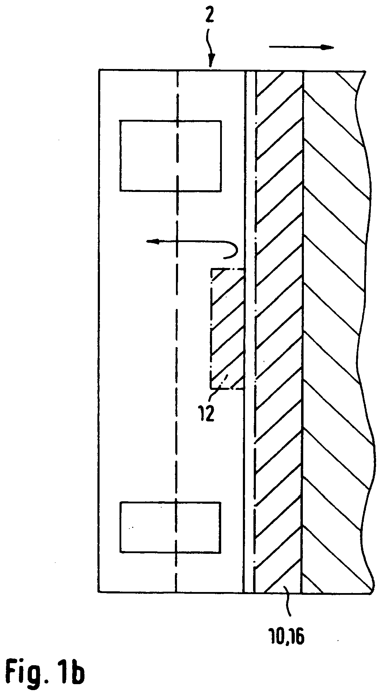

Von einem Hygieneartikel wird ein an den Hauptteil angefügter auf sich selbst gefalteter Materialabschnitt entlang eines Seitenlängsrandes des Hauptteils unter Zerstörung der Anfügung abgetrennt. Hierfür kann eine Klinge oder Schere Verwendung finden. Der den betreffenden Seitenteil bildende Materialabschnitt 2 wird gemäß Figuren 1a und 1b mit einem Längsseitenrand 4 an eine untere Klemme 6 des Zugprüfgeräts über seine gesamte Länge (in Längsrichtung des Inkontinenzartikels), mit der er zuvor an den Hauptteil angefügt war, fest eingespannt. Die untere Klemme 6 des Zugprüfgeräts 8 muss deshalb eine entsprechende Länge, zweckmäßigerweise eine Länge von 300 mm aufweisen. Am gegenüberliegenden freien Längsseitenrand 10 des abgetrennten Materialabschnitts 2, der dort einen Anfassbereich 12 bildet, wird die bewegbare Klemme 14 des Zugprüfgeräts 8 über eine Länge von 60 mm eingespannt. Bei einer Längserstreckung des Materialabschnitts von weniger als 160 mm wird der Materialabschnitt 2 am freien Längsseitenrand 10 über eine Länge von 30 mm an der beweglichen Klemme 14 eingespannt. Die nicht maßstabsgetreue Figur 1b zeigt den Materialabschnitt 2 in der gefalteten Konfiguration, wobei der in die Klemme 6 einspannbare Bereich 16 am Längsseitenrand 10 und der in der Klemme 14 einspannbare Anfassbereich 12 schraffiert dargestellt ist. Die Pfeile deuten die Zugrichtung des Zugprüfgeräts an. In Figur 1a ist ferner die Einspannlänge Lₛₚ angedeutet.

Durch gesteuerte Bewegung dieser bewegbaren Klemme 14 wird ein Zugprüfversuch durchgeführt.

### Prüfparameter:

- Prüfgeschwindigkeit der bewegbaren Klemme: 300 mm/min
- Einspannlänge: 10 mm
- Messweg: Länge der Quererstreckung des entfalteten Materialabschnitts
- Vorkraft: 0,01 N.

### Auswertung:

Das Prüfresultat des Zugprüfversuchs wird als in dem Materialabschnitt aufgetretene und zwischen den Klemmen ermittelte Zugkraft gerundet auf zwei Dezimalstellen in N angegeben. Es wird ein Kraft/Wegdiagramm erstellt.

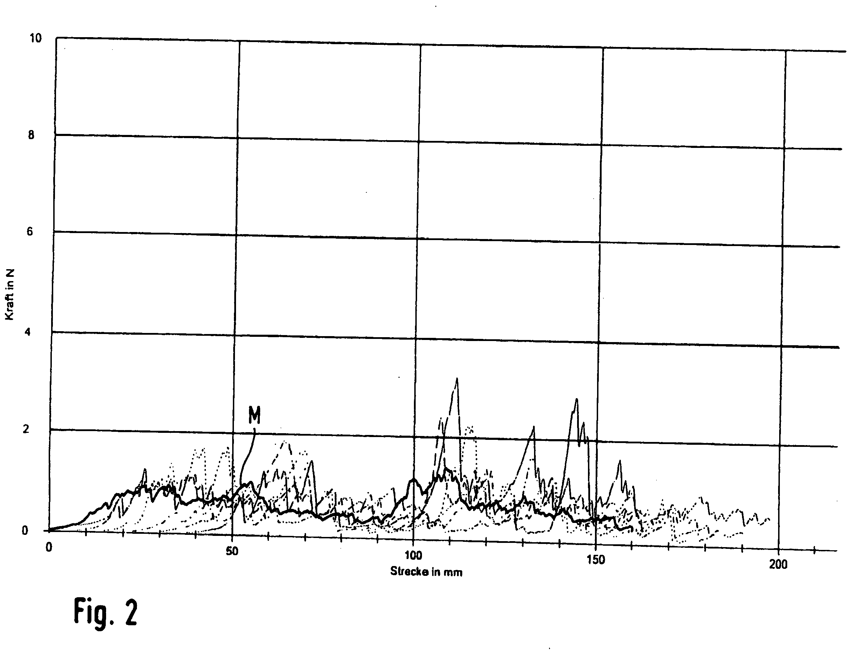

Ein solches Kraft/Wegdiagramm zeigt Figur 2. Es sind in Figur 2 die Ergebnisse von sechs Zugprüfversuchen und fett eine hieraus gemittelte Kurve M bei einem nachfolgend noch zu beschreibenden Inkontinenzartikel dargestellt. In der nachfolgenden Tabelle sind jeweils die Spitzenkräfte Fmax und die für einen jeweiligen Entfaltungsvorgang ermittelten Mittelwerte von Fmax und der Mittelwert X(n=6) aus den sechs Zugprüfversuchen angegeben. Von einer Kraftspitze Fmax wurde ausgegangen, wenn sich diese um 0,5 N von einem benachbarten Kraftminimum unterschied.

In der Tabelle außerdem angegeben ist die zum Öffnen erforderliche Arbeit in Nmm, die rechnerisch aus den ermittelten Zugkräften und dem Weg ermittelt wurde.

| Nr | Mittelwert Fₘₐₓ in N | Fₘₐₓ in N | W in Nmm |
|---|---|---|---|
| 1 | 1,78 | 3,24 | 95,69 |
| 2 | 1,41 | 2,41 | 91,63 |
| 3 | 1,61 | 2,25 | 89,91 |
| 4 | 1,16 | 1,39 | 62,66 |
| 5 | 1,12 | 1,62 | 60,84 |
| 6 | 1,69 | 2,86 | 95,40 |
| X(n=6) | 1,46 | 2,29 | 82, 69 |

Die Ergebnisse nach Figur 2 und der vorstehenden Tabelle wurden ermittelt bei der Entfaltung von Materialabschnitten, die in einem Rückbereich eines erfindungsgemäßen Hygieneartikels vorgesehen waren, der nachfolgend noch beschrieben wird, wobei diese Materialabschnitte zugleich versteifende Verschlusselemente aufweisen.

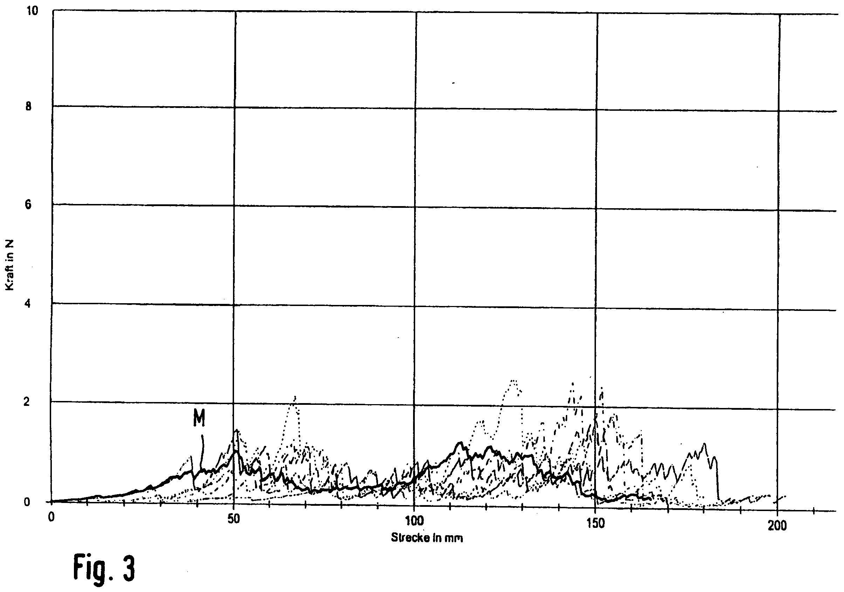

Entsprechende Messungen wurden an in einem Vorderbereich des Hygieneartikels vorgesehenen Materialabschnitten einer geringeren Längserstreckung und ohne versteifende Verschlusselemente vorgenommen. Die Ergebnisse sind in Figur 3 und in der nachfolgenden Tabelle in entsprechender Weise dargestellt.

| Nr | Mittelwert Fₘₐₓ in N | Fₘₐₓ in N | W in Nmm |
|---|---|---|---|
| 1 | 1,05 | 1,48 | 64,67 |
| 2 | 1,20 | 1,77 | 73,73 |
| 3 | 1,52 | 2,54 | 105,45 |
| 4 | 1,73 | 2,49 | 93,93 |
| 5 | 1,32 | 1,88 | 59,04 |
| 6 | 1,10 | 1,30 | 66,19 |
| X(n=6) | 1,32 | 1,91 | 77,17 |

Weitere Merkmale, Einzelheiten und Vorteile der Erfindung ergeben sich aus den beigefügten Patentansprüchen, der zeichnerischen Darstellung und nachfolgenden Beschreibung einer bevorzugten Ausführungsform der Erfindung. In der Zeichnung zeigt:
Figur 1a eine schematische Darstellung der Einspannung der eines Materialabschnitts in ein Zugprüfgerät;
Figur 1b eine nicht maßstabsgetreue Darstellung eines Materialabschnittes in der gefalteten Konfiguration
Figuren 2,3 bei einem Zugprüfversuch ermittelte Kraft/Weg-Diagramme;
Figur 4 eine Draufsicht auf einen erfindungsgemäßen Hygieneartikel in schematischer Darstellung;
Figur 5 eine Schnittansicht des Hygieneartikels nach Figur 4 (A-A);
Figur 6 eine Figur 5 entsprechende Schnittansicht mit eingeschlagenem Materialabschnitt;
Figur 7 eine vergrößerte Darstellung eines Materialabschnitts in gefalteter Konfiguration;
Figur 8 eine schematische Schnittansicht eines gefalteten Materialabschnitts.

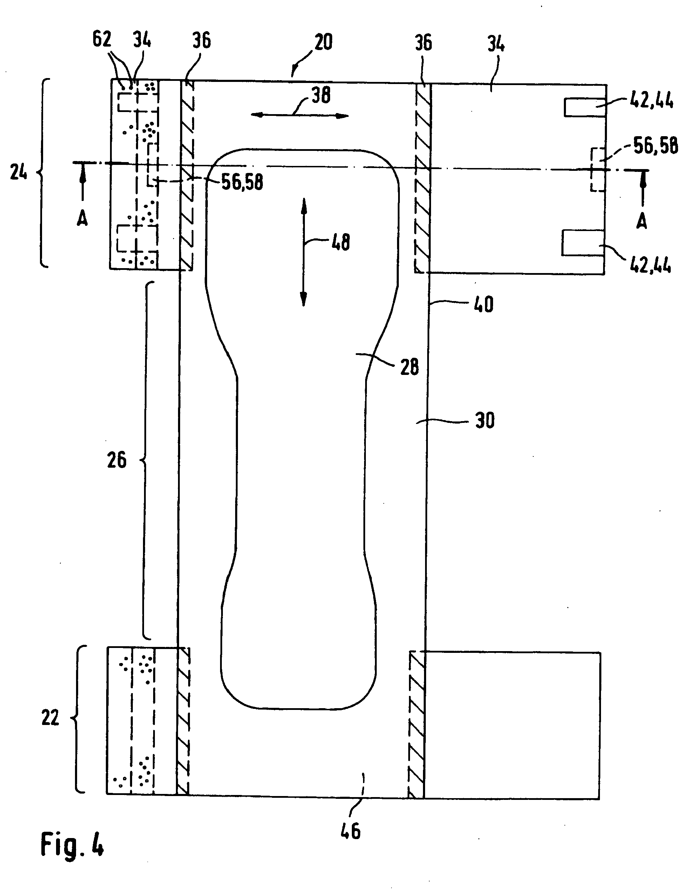

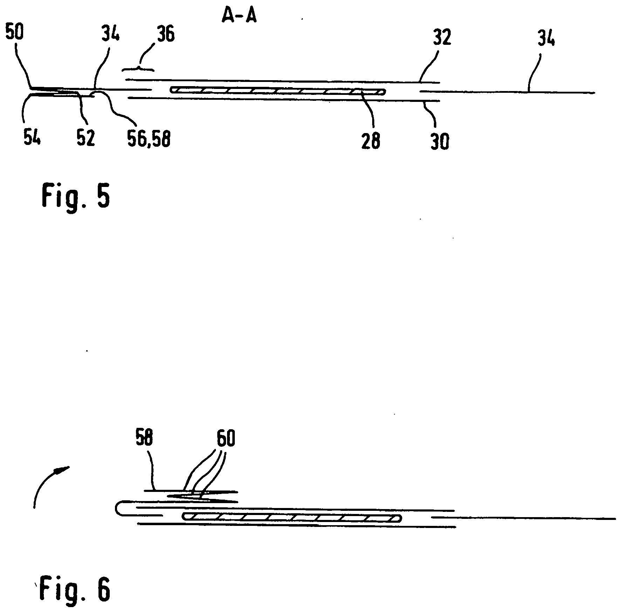

Der erfindungsgemäße Inkontinenzartikel ist in den Figuren 4 bis 7 schematisch dargestellt. Er umfasst einen insgesamt mit dem Bezugszeichen 20 bezeichneten Hauptteil, der häufig auch als Chassis bezeichnet wird. Der Hauptteil 20 umfasst einen Vorderbereich 22, einen Rückbereich 24 und einen dazwischen liegenden Schrittbereich 26, der zwischen den Beinen eines Benutzers zu liegen kommt, wenn der Hygieneartikel an einen Benutzer angelegt ist. Der Hauptteil 20 umfasst einen Saugkörper 28, der zur Aufnahme und dauerhaften Speicherung von Körperflüssigkeiten in geeigneter Weise dimensioniert ist. Der Saugkörper ist von einer flüssigkeitsundurchlässigen Schicht 30 unterfangen, welche auch die äußere Sichtseite des Inkontinenzartikels bilden kann. Oberhalb des Saugkörpers 28 kann ein flüssigkeitsdurchlässiges Topsheet 32 vorgesehen sein, welches in der Schnittansicht der Figur 5 dargestellt ist.

Im Rückbereich 24 ist beidseits an den Hauptteil 20 ein Seitenklappen oder Seitenabschnitte bildender Materialabschnitt 34 an einen Längsrandabschnitt 36 des Hauptteils 20 angefügt.

Figur 4 zeigt den in der Zeichnung rechten Materialabschnitt 34 im vollständig entfalteten Zustand. Er erstreckt sich in Querrichtung 38 über den betreffenden Längsrand 40 des Hauptteils 20 hinaus in Hüftumfangsrichtung. Der Materialabschnitt trägt zwei Verschlusselemente 42 in Form von auf sich selbst gefalteten und zum bestimmungsgemäßen Gebrauch entfaltbaren Verschlusstapes 44, die zum Schließen des Hygieneartikels mit einer Außenseite 46 des Vorderbereichs 22 des Hauptteils 20 lösbar haftend zusammenwirken.

Der jeweilige Materialabschnitt 34 im Rückbereich 24 des Hauptteils ist, wie aus der Figur 4 oben links und der Schnittdarstellung nach Figur 5 ersichtlich, entlang dreier in Längsrichtung 48 erstreckter Falzlinien 50, 52, 54 leporelloförmig auf sich selbst gefaltet.

Ein freier Längsrandabschnitt 56 des jeweiligen Materialabschnitts 34 bildet einen Anfassbereich 58 zum manuellen Ergreifen des gefalteten Materialabschnitts 34, um diesen zu entfalten. Bei der Abgabe an den Letztverbraucher sind die jeweiligen Materialabschnitte 34 ausgehend von der Darstellung nach Figur 5 nach innen geschlagen in die in Figur 6 angedeutete Position. Man erkennt, dass der freie Längsrandabschnitt 56 und damit der Anfassbereich 58 des Materialabschnitts 34 von einer Längsmittelachse des Inkontinenzartikels weg nach außen gewandt ist, so dass bei auf einer Unterlage ausgebreitetem Hygieneartikel der Anfassbereich 58 bequem mit der linken Hand eines Benutzers von links und ein entsprechend positionierter Anfassbereich des anderen Materialabschnitts mit der rechten Hand des Benutzers von rechts ergriffen werden kann. Es wäre auch denkbar, dass die nach innen auf die Oberseite des Hauptteils gefalteten Materialabschnitte einander teilweise überlappen, wobei es sich als vorteilhaft erweist, wenn in dieser überlappten Konfiguration die jeweiligen Anfassbereiche der Materialabschnitte zugleich ergreifbar sind.

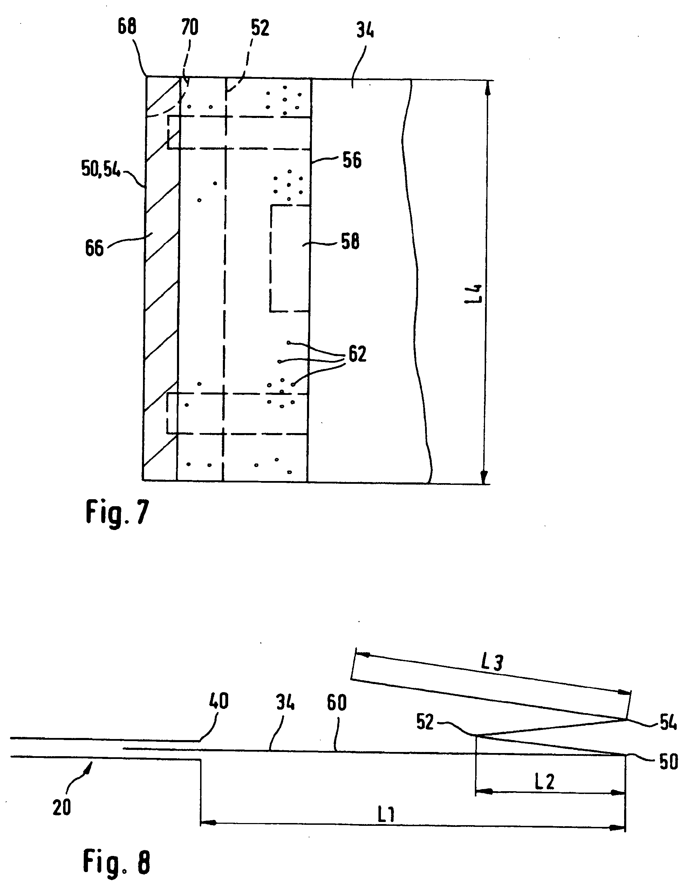

Die aufeinander gefalteten Teilabschnitte 60 der Materialabschnitte 34 sind in der gefalteten Konfiguration durch punktförmige durch Ultraschallschweißung erzeugte Fügestellen 62 mit einem Durchmesser von 0,35 mm und einer Fläche von 0,0962 mm², die in Figur 4 angedeutet sind, lösbar aneinander fixiert. Es hat sich gezeigt, dass diese lösbare Fixierung so gestaltet sein kann, dass sich durch einmaliges Ziehen an dem jeweiligen Anfassbereich 58 der Materialabschnitt 34 vollständig entfalten lässt, wobei alle Fügestellen 62 gelöst oder aufgetrennt werden. Hierbei erweist es sich als vorteilhaft, wenn in einem Abstand von wenigstens 5 mm, vorzugsweise von wenigstens 8 mm und weiter vorzugsweise von wenigstens 10 mm von den Falzlinien 50, 54 entfernt keine Fügestelle der Teilabschnitte 60 vorgesehen ist. Dieser fügestellenfreie Bereich ist in der Figur 7 mit Bezugszeichen 66 bezeichnet. Ausgehend von einem vom Anfassbereich 58 am weitesten entfernten Punkt 68 auf der Falzlinie 50, 54 ist in einem schematisch angedeuteten Umkreis 70 keine Fügestelle vorgesehen. Diese sehr weit von dem Anfassbereich 58 entfernten und in der Nähe einer Falzlinie angeordneten Bereiche sind im Hinblick auf eine vollständige Entfaltung, d. h. eine Auftrennung sämtlicher Fügestellen besonders kritisch. Daher erweist es sich als vorteilhaft, wenn in diesen kritischen Bereichen nur wenige oder vorzugsweise keine Fügestellen vorgesehen sind, so dass die gefalteten Materialabschnitte in einem Zug, also ohne mehrfach Nachfassen oder ohne mehrfach ruckartig an dem Anfassbereich 58 ziehen zu müssen, lösbar sind bzw. entfaltbar sind.

Anhand der Figur 8 werden die Abmessungen der Teilabschnitte 60 des Materialabschnitts 34 deutlich. Die Erstreckung L1 in Querrichtung 38 vom Längsrand 40 des Hauptteils 20 bis zur Falzlinie 50 beträgt 90 mm. Die Erstreckung L2 zwischen den Falzlinien 50 - 52 - 54 beträgt jeweils 35 mm, und die Erstreckung L3 bis zum freien Ende beträgt 65 mm. Dies Gesamterstreckung in Querrichtung des entfalteten Materialabschnitts beträgt daher in Querrichtung 38 225 mm. Die Längserstreckung L4 beträgt 260 mm.

Wie aus Figur 4 ersichtlich ist, sind im Vorderbereich 22 des Hygieneartikels ebenfalls auf sich selbst gefaltete Materialabschnitte 34 mit einer Längserstreckung L4 von nur 200 mm und einer Quererstreckung von 225 mm vorgesehen, die jedoch keine Verschlusselemente aufweisen, welche die Materialabschnitte versteifen und deshalb auch zum Lösen benachbart angeordneter Fügestellen aufgrund ihrer Steifigkeit beitragen. In unmittelbarer Nähe zu solchen versteifenden Verschlusselementen können nämlich Fügestellen oder Fügebereiche vorgesehen werden, die aufgrund der Versteifung gut lösbar sind.

Wie bereits erwähnt, wurden die Messergebnisse nach Tabelle 1 und Figur 2 bei Entfaltungsvorgängen der Materialabschnitte 34 im Rückbereich 24 und die Messergebnisse nach Tabelle 2 und Figur 3 bei Entfaltungsvorgängen der Materialabschnitte 34 im Vorderbereich 22 ermittelt.

## Patentansprüche

1. Absorbierender Inkontinenzartikel mit einem Hauptteil (20), bestehend aus einem Vorderbereich (22), einem Rückbereich (24) und einem in Längsrichtung- (48) dazwischen liegenden zwischen den Beinen eines Benutzers zu liegen kommenden Schrittbereich (26), wobei der Hauptteil (20) einen Saugkörper (28) umfasst, und mit beidseits an den Rückbereich (24) angefügten Materialabschnitten (34, 2), welche sich in Querrichtung (38) über seitliche Längsränder (40) des Hauptteils (20) hinaus erstrecken und den Vorderbereich (22) und den Rückbereich (24) im angelegten Zustand des Artikels miteinander verbinden, wobei diese Materialabschnitte (34, 2) wenigstens um eine in Längsrichtung (48) verlaufende Falzlinie (50, 52, 54) auf sich selbst gefaltet sind und aufeinander gefaltete und flächenhaft aneinander liegende Teilabschnitte (60) der Materialabschnitte (34, 2) in dieser gefalteten Konfiguration an Fügestellen (62) oder Fügebereichen lösbar aneinander fixiert sind, wobei ein jeweiliger so gefalteter Materialabschnitt: (34, 2) einen Anfassbereich (58, 12) zum Entfalten des Materialabschnitts (34, 2) aufweist und wobei die beidseits vorgesehenen Materialabschnitte (34, 2) eines vor der Ingebrauchnahme zusammengefalteten Artikels auf die körperzugewandte Seite des Hauptteils (20) eingeschlagen sind, **dadurch gekennzeichnet, dass** die Anzahl, die Verteilung oder der Flächenanteil der Fügestellen (62) oder die Haftkraft der lösbar aneinander gefügten Teilabschnitte (60) so gewählt ist, dass die lösbare Fixierung an sämtlichen Fügestellen (62) oder Fügebereichen beim Entfalten durch einmaliges Ziehen an einem jeweiligen Anfassbereich (58, 12) der Materialabschnitte (34, 2) auftrennbar ist, und wobei die beidseits an den Rückbereich (24) angefügten Materialabschnitte jeweils Verschlusselemente (42) aufweisen, die mit einem Auftreffbereich am Hauptteil (20) lösbar haftend oder klebend zusammenwirken, und dass die lösbare Fixierung der aufeinander gefalteten Teilabschnitte (60) der Materialabschnitte (34, 2) aneinander durch mehrere im wesentlichen punktförmige Fügestellen (62) ausgebildet ist und dass die lösbare Fixierung der aufeinander gefalteten Teilabschnitte (60) der Materialabschnitte (34, 2) aneinander durch thermisch oder durch Ultraschall erzeugte Fügestellen (62) ausgebildet ist.

2. Inkontinenzartikel nach Anspruch 1, **dadurch gekennzeichnet, dass** ein gefalteter Materialabschnitt (34, 2) um zwei, drei oder vier Faltlinien (50, 52, 54) auf sich selbst gefaltet ist.

3. Inkontinenzartikel nach Anspruch 1 oder 2, **dadurch gekennzeichnet, dass** vor der Ingebrauchnahme die jeweiligen Anfassbereiche (58, 12) in Querrichtung (38) nach außen also voneinander weg gewandt sind, so dass sie auf bequeme Weise mit der linken Hand eines Benutzers von links und mit der rechten Hand von rechts ergreifbar sind.

4. Inkontinenzartikel nach einem oder mehreren der vorstehenden Ansprüche, **dadurch gekennzeichnet**, durch einen Bereich der Materialabschnitte (34, 2) in gefalteter Konfiguration, in dem die Anzahl oder der Flächenanteil der Fügestellen (62) oder die Haftkraft der lösbar aneinander gefügten Teilabschnitte der Materialabschnitte mit der Entfernung von dem Anfassbereich in Längsrichtung (48) abnimmt.

5. Inkontinenzartikel nach einem oder mehreren der vorstehenden Ansprüche, **gekennzeichnet durch** einen Bereich der Materialabschnitte (34, 2) in gefalteter Konfiguration, in dem die Anzahl oder der Flächenanteil der Fügestellen (62) oder die Haftkraft der lösbar aneinander gefügten Teilabschnitte (60) der Materialabschnitte mit der Entfernung von dem Anfassbereich (58, 12) in Querrichtung (38) abnimmt.

6. Inkontinenzartikel nach einem oder mehreren der vorstehenden Ansprüche, **dadurch gekennzeichnet, dass** in einem Umkreis (70) von 1,5 cm von einem vom Anfassbereich (58, 12) am weitesten entfernten Punkt (68) der am weitesten entfernten Falzlinie (50, 54) die aneinanderliegenden Teilabschnitte (60) nicht aneinander gefügt sind.

7. Inkontinenzartikel nach einem oder mehreren der vorstehenden Ansprüche, **dadurch gekennzeichnet, dass** in einem Abstand von 5 mm, insbesondere von 8 mm und weiter insbesondere von 10 mm von der vom Anfassbereich (58, 12) am weitesten entfernten Falzlinie (50, 54) die aneinanderliegenden Teilabschnitte (60) nicht aneinander gefügt sind.

8. Inkontinenzartikel nach einem oder mehreren der vorstehenden Ansprüche, **dadurch gekennzeichnet, dass** die flächenhafte Erstreckung der aufeinandergefalteten und aneinander anliegenden Teilabschnitte (60) durch eine in Längsrichtung (48) verlaufende Gerade (52) in zwei annähernd gleiche Hälften teilbar ist und dass die Anzahl oder der Flächenanteil der Fügestellen (62) oder die Haftkraft der lösbar aneinander gefügten Teilabschnitte (60) in diesen zwei Hälften unterschiedlich ist.

9. Inkontinenzartikel nach Anspruch 8, **dadurch gekennzeichnet, dass** die Anzahl oder der Flächenanteil der Fügestellen oder die Haftkraft der lösbar aneinander gefügten Teilabschnitte (60) in der dem Anfassbereich (58, 12) in Querrichtung (38) zugewandten Hälfte größer ist als in der dem Anfassbereich in Querrichtung abgewandten Hälfte.

10. Inkontinenzartikel nach einem oder mehreren der vorstehenden Ansprüche, **dadurch gekennzeichnet, dass** die Erstreckung eines Materialabschnitts (34, 2) in Längsrichtung (48) im Bereich der Anfügung an den Hauptteil (20) wenigstens 10 cm, insbesondere wenigstens 14 cm, insbesondere wenigstens 18 cm und weiter insbesondere wenigstens 22 cm beträgt und/oder dass die Erstreckung eines Materialabschnitts (34, 2) im entfalteten Zustand in Querrichtung (38) über den Längsrand (40) des Hauptteils (20) hinaus wenigstens 10 cm, insbesondere wenigstens 15 cm, insbesondere wenigstens 18 cm, insbesondere wenigstens 22 cm beträgt.

11. Inkontinenzartikel nach einem oder mehreren der vorstehenden Ansprüche, **dadurch gekennzeichnet, dass** die Materialabschnitte (34, 2) Verschlusselemente (42) aufweisen, die vorzugsweise ihrerseits in einer gefalteten, zum Gebrauch entfaltbaren Konfiguration an den Materialabschnitten angeordnet sind.

12. Inkontinenzartikel nach einem oder mehreren der vorstehenden Ansprüche, **dadurch gekennzeichnet, dass** die Fügestellen (62) oder Fügebereiche beim Entfalten der Materialabschnitte (34, 2) durch Ziehen an dem jeweiligen Anfassbereich (58, 12) eine über den Entfaltungsvorgang gemittelte Spitzenkraft von höchstens 2,5 N, insbesondere von höchstens 2,4 N, insbesondere von höchstens 2,3 N, insbesondere von höchstens 2,2 N, insbesondere von höchstens 2,1 N und weiter insbesondere von höchstens 2,0 N entgegensetzen.

13. Inkontinenzartikel nach einem oder mehreren der vorstehenden Ansprüche, **dadurch gekennzeichnet, dass** die Fügestellen (62) oder Fügebereiche beim Entfalten der Materialabschnitte (34, 2) durch Ziehen an dem jeweiligen Anfassbereich (58, 12) eine über sechs Entfaltungsvorgänge gemittelte Spitzenkraft von höchstens 2,0 N, insbesondere von höchstens 1,8 N, insbesondere von höchstens 2,3 N, insbesondere von höchstens 1,7 N, insbesondere von höchstens 1,6 N und weiter insbesondere von höchstens 1,5 N entgegensetzen.

14. Inkontinenzartikel nach einem oder mehreren der vorstehenden Ansprüche, **dadurch gekennzeichnet, dass** die beim Entfalten eines Materialabschnitts (34, 2) in einem Zug erforderliche Arbeit gemittelt über sechs Entfaltungsvorgänge höchstens 120 Nmm, insbesondere höchstens 115 Nmm, insbesondere höchstens 110 Nmm, insbesondere höchstens 105 Nmm, insbesondere höchstens 100 Nmm, insbesondere höchstens 95, insbesondere höchstens 90 Nmm beträgt.

## Claims

1. An absorbent incontinence article with a main body portion (20), consisting of a front region (22), a back region (24) and a crotch region (26) lying therebetween in a longitudinal direction (48) finally positioned between the legs of a wearer, where the main body portion (20) comprises an absorbent core (28), and with material sections (34, 2) attached on both sides to the back region (24) which extend in the transverse direction (38) beyond lateral longitudinal edges (40) of the main body portion (20) and connect the front region (22) and the back region (24) of the main body portion (20) when the article is worn, wherein these material sections (34, 2) are folded onto themselves along at least one fold line (50, 52, 54) running in the longitudinal direction (48) and wherein partial sections (60) of those material sections (34, 2) folded onto each other and lying face-to-face against each other are releasably attached to one another at affixation points (62) or affixation areas, wherein a folded respective such material section (34, 2) comprises a gripping area (58, 12) for unfolding the material section (34), and wherein the material sections (34, 2) provided on both sides of an article folded prior to use are folded inwards onto the body-facing side of the main body portion (20), **characterized in, that** the number, the arrangement or the relative surface area of the affixation points (62) or the adherent strength of the releasably attached partial sections (60) of the material sections is selected such that the releasable attachment at all those affixation points (62) or affixation areas can be undone when unfolding by a single pull on a respective gripping area (58, 12) of the material section (34, 2), and wherein the material sections attached on both sides to the back region (24) each comprising closure elements (42) which interact in a releasable adherent or adhesive manner with a landing region on the main body portion (20), and **characterized in that** the releasable attachment to each other of the partial sections (60) of the material sections (34, 2) folded over themselves is formed by a plurality of substantially punctiform affixation points (62) and that the releasable attachment to each other of the partial sections (60) of the material sections (34, 2) folded over themselves is formed by thermally or ultrasonically formed affixation points (62).

2. The incontinent article of claim 1, **characterized in that** a folded material section (34, 2) is folded onto itself along two, three or four fold lines (50, 52, 54).

3. The incontinent article of claim 1 or 2, **characterized in that** the respective gripping areas (58, 12) are oriented outward away from each other in the transverse direction (38) before use so that a user can grasp the gripping areas comfortably with the left hand from the left and the right hand from the right.

4. The incontinent article according to one or more of the preceding claims, **characterized by** a region of the material sections (34, 2) in the folded configuration in which the number or the relative surface area of the affixation points (62) or the adherent strength of the releasably attached partial sections of the material sections decreases with distance from the gripping area in the longitudinal direction (48).

5. The incontinent article according to one or more of the preceding claims, **characterized by** a region of the material sections (34, 2) in the folded configuration in which the number or the relative surface area of the affixation points (62) or the adherent strength of the releasably attached partial sections (60) of the material sections decreases with distance from the gripping area (58, 12) in the transverse direction (38).

6. The incontinent article according to one or more of the preceding claims, **characterized in that** in a compass (70) of 1,5 cm around a point on a most distant fold line farthest removed from the gripping area (58, 12) the adjoining partial sections (60) are not attached to each other.

7. The incontinent article according to one or more of the preceding claims, **characterized in that** within a distance of 5 mm, particularly of 8 mm and further particularly of 10 mm from the fold line (50, 54) farthest removed from the gripping area (58, 12), the adjoining partial sections (60) are not attached to each other.

8. The incontinent article according to one or more of the preceding claims, **characterized in that** an area extension of the partial sections (60) folded onto each other and adjoining one another can be divided into two approximately equal halves by a straight line (52) running in the longitudinal direction (48) and that the number or the relative surface area of the affixation points (62) or the adherent strength of the releasably attached partial sections (60) is different in the two halves.

9. The incontinent article of claim 8, **characterized in that** the number or the relative surface area of the affixation points or the adherent strength of the releasably attached partial sections (60) in the half facing the gripping area (58, 12) in the transverse direction (38) is greater than in the half facing away from the gripping area in the transverse direction.

10. The incontinent article according to one or more of the preceding claims, **characterized in that** the extension of a material section (34, 2) in the longitudinal direction (48) in the area of attachment to the main body portion (20) measures at least 10 cm, particularly at least 14 cm, particularly at least 18 cm and further particularly at least 22 cm and/or that the extension of a material section (34, 2) in the unfolded state in the transverse direction (38) beyond the longitudinal edge (40) of the main body portion (20) measures at least 10 cm, particularly at least 15 cm, particularly at least 18 cm, particularly at least 22 cm.

11. The incontinent article according to one or more of the preceding claims, **characterized in that** the material sections (34, 2) have closure elements (42) which are preferably arranged at the material sections in a folded configuration, ready to be unfolded for use.

12. The incontinent article according to one or more of the preceding claims, **characterized in that** the affixation points (62) or affixation areas offer a peak resistance averaged over the unfolding process of 2,5 N maximum, particularly 2,4 N maximum, particularly 2,3 N maximum, particularly 2,2 N maximum, particularly 2,1 N maximum, further particularly 2,0 N maximum when the material sections (34, 2) are unfolded by pulling on the respective gripping area (58, 12).

13. The incontinent article according to one or more of the preceding claims, **characterized in that** the affixation points (62) or affixation areas offer a peak resistance averaged over six unfoldings of 2,0 N maximum, particularly 1,8 N maximum, particularly 2,3 N maximum, particularly 1,7 N maximum, particularly 1,6 N maximum, particularly 1,5 N maximum when the material sections (34, 2) are unfolded by pulling on the respective gripping area (58, 12).

14. The incontinent article according to one or more of the preceding claims, **characterized in that** the work required when unfolding a material section (34, 2) in one pull averaged over six unfoldings measures 120 Nmm maximum, particularly 115 Nmm maximum, particularly 110 Nmm maximum, particularly 105 Nmm maximum, particularly 100 Nmm maximum, particularly 95 Nmm maximum, particularly 90 Nmm.

## Revendications

1. Article absorbant pour personnes incontinentes comportant une partie principale (20) composée d'une zone avant (22), d'une zone arrière (24) et d'une zone d'entrejambe (26) située entre les deux dans le sens longitudinal (48) et venant entre les jambes d'un utilisateur, la partie principale (20) comprenant un corps absorbant (28), et comportant des segments de matière (34, 2) assemblés à la zone arrière (24), lesquels segments s'étendent dans le sens transversal (38) au-delà des bords longitudinaux latéraux (40) de la partie principale (20) et, une fois l'article mis en place, relient entre elles la zone avant (22) et la zone arrière (24) de la partie principale (20), les segments de matière (34, 2) étant pliés sur eux-mêmes autour d'au moins une ligne de pliage (50, 52, 54) s'étendant dans le sens longitudinal (48), et des segments partiels (60) des segments de matière (34, 2) repliés sur eux-mêmes et reposant les uns sur les autres dans cette configuration pliée étant fixés de façon amovible par points d'assemblage (62) ou par des zones d'assemblage, un respective segment de matière ainsi plié (34, 2) présentant une zone de préhension (58, 12) pour déplier le segment de matière (34, 2), les segments de matière (34, 2) prévus de chaque côté d'un article plié avant utilisation étant pliés sur le côté orienté vers le corps de la partie principale (20), **caractérisé en ce que** le nombre ou l'arrangement, ou surface relative des points d'assemblage (62), ou bien l'adhérence des segments partiels (60) assemblés les uns aux autres de façon amovible, est selecté de sorte que la fixation amovible au niveau de l'ensemble des points d'assemblage (62) ou des zones d'assemblage pouvant être séparée lors du dépliage en tirant une fois au niveau de la zone de préhension correspondante (58, 12) des segments de matière (34, 2) et les segments de matière joints de chaque côté de la zone arrière présentant des éléments de fermeture (42) qui coopèrent de façon amovible par adhérence ou collage avec une zone d'impact au niveau de la partie principale (20) et **en ce que** la fixation amovible des segments partiels (60) repliés sur eux-mêmes des segments de matière (34, 2) est réalisée par plusieurs points d'assemblage (62) essentiellement en forme de points et **en ce que** la fixation amovible des segments partiels (60) repliés sur eux-mêmes des segments de matière (34, 2) est réalisée par des points d'assemblage (62) produits thermiquement ou par ultrasons.

2. Article pour personnes incontinentes selon la revendication 1, **caractérisé en ce qu'**un segment de matière plié (34, 2) est plié sur lui-même autour de deux, trois ou quatre lignes de pliage (50, 52, 54).

3. Article pour personnes incontinentes selon la revendication 1 ou 2, **caractérisé en ce que**, avant l'utilisation, chacune des zones de préhension (58, 12) est orientée vers l'extérieur dans le sens transversal (38) de sorte qu'elles puissent être aisément saisies par la gauche par la main gauche d'un utilisateur et par la droite par la main droite d'un utilisateur.

4. Article pour personnes incontinentes selon une ou plusieurs des revendications précédentes, **caractérisé par** une zone des segments de matière (34, 2) en configuration pliée, dans laquelle le nombre ou la surface des points d'assemblage (62), ou bien l'adhérence des segments partiels assemblés les uns aux autres de façon amovible des segments de matière, diminue à mesure que l'on s'éloigne de la zone de préhension dans le sens longitudinal (48).

5. Article pour personnes incontinentes selon une ou plusieurs des revendications précédentes, **caractérisé par** une zone des segments de matière (34, 2), en configuration pliée, dans laquelle le nombre ou la surface des points d'assemblage (62), ou bien l'adhérence des segments partiels (60) assemblés les uns aux autres de façon amovible des segments de matière, diminue à mesure que l'on s'éloigne de la zone de préhension (58, 12) dans le sens transversal (38).

6. Article pour personnes incontinentes selon une ou plusieurs des revendications précédentes, **caractérisé en ce que**, dans un rayon (70) de 1,5 cm à partir d'un point (68) le plus éloigné de la zone de préhension (58, 12) de la ligne de pliage la plus éloignée (50, 54), les segments partiels (60) reposant les uns sur les autres ne sont pas assemblés les uns aux autres.

7. Article pour personnes incontinentes selon une ou plusieurs des revendications précédentes, **caractérisé en ce que**, à une distance de 5 mm, en particulier de 8 mm, et plus particulièrement à une distance de 10 mm de la ligne de pliage (50, 54) la plus éloignée de la zone de préhension (58, 12), les segments partiels (60) reposant les uns sur les autres ne sont pas assemblés les uns aux autres.

8. Article pour personnes incontinentes selon une ou plusieurs des revendications précédentes, **caractérisé en ce que** la surface des segments partiels (60) repliés sur eux-mêmes et reposant les uns sur les autres peut être divisée en deux moitiés quasi-égales par une droite (52) s'étendant dans le sens longitudinal (48) et **en ce que** le nombre ou la surface des points d'assemblage (62) ou bien l'adhérence des segments partiels (60) assemblés les uns aux autres de façon amovible est différent(e) dans ces deux moitiés.

9. Article pour personnes incontinentes selon la revendication 8, **caractérisé en ce que** le nombre ou la surface des points d'assemblage ou bien l'adhérence des segments partiels (60) assemblés les uns aux autres de façon amovible dans la moitié orientée vers la zone de préhension (58, 12) dans le sens transversal (38) est plus grand(e) que dans la moitié orientée à l'opposé de la zone de préhension dans le sens transversal.

10. Article pour personnes incontinentes selon une ou plusieurs des revendications précédentes, **caractérisé en ce que** l'étendue d'un segment de matière (34, 2) dans le sens longitudinal (48) dans la zone de l'assemblage à la partie principale (20) est égale à au moins 10 cm, en particulier au moins 14 cm, en particulier au moins 18 cm et plus particulièrement au moins 22 cm et/ou **en ce que** l'étendue d'un segment de matière (34, 2) à l'état déplié dans le sens transversal (38) au-delà du bord longitudinal (40) de la partie principale (20) est égale à au moins 15 cm, en particulier au moins 18 cm, et plus particulièrement au moins 22 cm.

11. Article pour personnes incontinentes selon une ou plusieurs des revendications précédentes, **caractérisé en ce que** les segments de matière (34, 2) présentent des éléments de fermeture (42) qui sont de préférence agencés, dans une configuration pliée mais dépliable en vue d'une utilisation, sur les segments de matière.

12. Article pour personnes incontinentes selon une ou plusieurs des revendications précédentes, **caractérisé en ce que** les points d'assemblage (62) ou les zones d'assemblage, lorsque l'on déplie les segments de matière (34, 2) en tirant au niveau de la zone de préhension correspondante (58, 12), présentent une force de pointe moyenneé par le processus de dépliage de 2,5 N maximum, en particulier de 2,4 N maximum, en particulier de 2,3 N maximum, en particulier de 2,2 N maximum, en particulier de 2,1 N maximum et plus particulièrement de 2,0 N maximum.

13. Article pour personnes incontinentes selon une ou plusieurs des revendications précédentes, **caractérisé en ce que** les points d'assemblage (62) ou zones d'assemblage, lorsque l'on déplie les segments de matière (34, 2) en tirant au niveau de la zone de préhension correspondante (58, 12), présentent une force de pointe moyenneé par six processus de dépliage de 2,0 N maximum, en particulier de 1,8 N maximum, en particulier de 2,3 N maximum, en particulier de 1,7 N maximum, en particulier de 1,6 N maximum et plus particulièrement de 1,5 N maximum.

14. Article pour personnes incontinentes selon une ou plusieurs des revendications précédentes, **caractérisé en ce que** la travail nécessaire lors du dépliage d'un segment de matière (34, 2) moyenneé par six processus de dépliage est égale à 120 Nmm maximum, en particulier à 115 Nmm maximum, en particulier à 110 Nmm maximum, en particulier à 105 Nmm maximum, en particulier à 100 Nmm maximum, en particulier à 95 Nmm maximum, en particulier à 90 Nmm maximum.
